# EUROPEAN PATENT SPECIFICATION

(11) **EP 2 271 214 B1**
(45) Date of publication and mention of the grant of the patent: **03.05.2017**
(21) Application number: 09724079.0
(22) Date of filing: 27.03.2009
(51) Int. Cl.: A61K 9/08, A61K 47/10

(54) **PHARMACEUTICAL SOLUTIONS AND METHOD FOR SOLUBILIZING THERAPEUTIC AGENTS**
PHARMAZEUTISCHE LÖSUNGEN UND VERFAHREN ZUR AUFLÖSUNG VON THERAPEUTIKA
SOLUTIONS PHARMACEUTIQUES ET PROCÉDÉ POUR SOLUBILISER DES AGENTS THÉRAPEUTIQUES

(30) Priority: 28.03.2008 US 40281 P
(43) Date of publication of application: 12.01.2011
(73) Proprietor: Particle Sciences, Inc., Bethlehem, PA 18017 (US)
(72) Inventor: GWOZDZ, Garry, Thomas, Nazareth PA 18064 (US); LOXLEY, Andrew, Philadelphia PA 19106 (US); MITCHNICK, Mark, East Hampton NY 11937 (US)
(74) Representative: O'Connell, Maura
(86) International application number: PCT/US2009/038518
(87) International publication number: WO 2009/120933

(56) References cited:
- WO-A1-00/01351
- WO-A1-03/020318
- WO-A1-03/057208
- US-A1- 2004 167 152
- US-A1- 2006 287 301
- US-A1- 2006 287 301
- US-A1- 2008 045 490
- US-A1- 2008 045 490
- US-A1- 2008 045 559
- US-B2- 6 696 413

## Description

### Background of the Invention

A vast number of potential therapeutic agents are discovered each year, many of which are water insoluble or poorly water soluble. For such hydrophobic compounds, direct injection may be impossible or highly dangerous, and can result in hemolysis, phlebitis, hypersensitivity, organ failure and/or death. Such compounds are termed by pharmacists as "lipophilic", "hydrophobic", or in their most insoluble form, "amphiphobic".

A few examples of therapeutic agents in these categories are ibuprofen, diazepam, griseofulvin, cyclosporin, cortisone, proleukin, etoposide and paclitaxel. (Kagkadis et al. PDA J. Pharm. Sci. Tech. 1996 50(5):317-323; Dardel Anaesth. Scand. 1976 20:221-24; Sweetana and Akers PDA J. Pharm. Sci. Tech. 1996 50(5):330-342).

Administration of chemotherapeutic agents is particularly problematic. Most of these agents are poorly soluble and thus are difficult to deliver in aqueous solvents and to supply at therapeutically effective levels. Further, water-soluble, chemotherapeutic agents are generally taken up by both cancer and non-cancer cells, making such agents non-specific and oftentimes unacceptably toxic.

For therapeutic agents that cannot be formulated as an aqueous solution, emulsions have oftentimes provided a cost-effective and therapeutically acceptable alternative. However, it is difficult to render emulsions sterile and/or endotoxin free for intravenous injection. Oils typically used for pharmaceutical emulsions include saponifiable oils from the family of triglycerides, for example, soybean oil, sesame seed oil, cottonseed oil, safflower oil and the like (Hansrani, et al. J. Parenter. Sci. Technol. 1983 37:145-150). One or more surfactants are used to stabilize the emulsion, and excipients are added to render the emulsion more biocompatible, stable and less toxic. Lecithin from egg yolks or soybeans is a commonly used surfactant. Sterile manufacturing can be accomplished by sterilization of all the components before manufacture, followed by aseptic technique in all stages of manufacture. Improved ease of manufacture and assurance of sterility is obtained by terminal sterilization following sanitary manufacture, either by heat or by filtration. However, terminal sterilization by heat or filtration treatments is not suitable for all emulsions.

Vitamin E emulsions have been disclosed. For example, injectable vitamin E emulsions are described by Hidiroglou and Karpinski (Brit. J. Nutrit. 1988 59:509-518) for dietary supplementation in sheep and for research on the pharmacokinetics of vitamin E and its derivatives. An injectable form of vitamin E for mice was prepared by Kato et al. (Chem. Pharm. Bull. 1993 41(3):599-604). Micellar solutions were formulated with TWEEN 80, BRIJ 58 and HCO-60. Isopropanol was used as a co-solvent, and was then removed by vacuum evaporation; the residual oil glass was then taken up in water with vortexing as a micellar suspension. An emulsion was also prepared by dissolving vitamin E with soy phosphatidycholine (lecithin) and soybean oil. Water was added and the emulsion prepared with sonication. Ethanol-free emulsions of alpha-tocopherol, stabilized by biocompatible surfactants, as a vehicle or carrier for therapeutic drugs is also disclosed in U.S. Patent Nos. 6,667,048 and 6,660,286.

E-Ferol, a vitamin E emulsion for vitamin E supplementation and therapy in neonates was also disclosed by Alade et al. (Pediatrics (1986) 77(4):593-597). The surfactant mixture used to emulsify the 25 mg/mL vitamin E in E-Ferol was composed of 9% TWEEN 80 and 1% TWEEN 20. However, this supplement was not safe.

An alternative means of solubilizing low solubility compounds is direct solubilization in a non-aqueous milieu, for example, alcohols (such as ethanol), dimethylsulfoxide, and/or triacetin. For example, WO 95/11039 describes the use of vitamin E (100 mg), lecithin (20 mg), ethanol (100 mg) and EUTANOL (500 mg) as an injectable formulation of the immunosuppressant molecule cyclosporine (50 mg). U.S. Patent No. 5,689,846 discloses various alcohol solutions of paclitaxel. U.S. Patent No. 5,573,781 discloses the dissolution of paclitaxel in ethanol, butanol and hexanol and an increase in the antitumor activity of paclitaxel when delivered in butanol and hexanol as compared to ethanol.

WO 95/31217 discloses that tocopherols can be used as solvents and/or emulsifiers of drugs that are substantially insoluble in water, in particular for the preparation of topical formulations. The use of vitamin E-TPGS as an emulsifier in formulations containing high levels of α-tocopherol is mentioned and formulations for topical administration composed of a lipid layer (α-tocopherol), the drug and vitamin E-TPGS as an emulsifier in quantities of less than 25% w/w of the formulation.

WO 97/03651 discloses lipid vehicle drug delivery compositions that contain at least five ingredients: a therapeutic drug, vitamin E, an oil in which the drug and vitamin E are dissolved, a stabilizer (either phospholipid, a lecithin, or a poloxamer which is a polyoxyethylene-polyoxypropylene copolymer) and water.

Similarly, U.S. Patent No. 6,962,691 teaches topical compositions composed of at least ten ingredients: alendronate sodium, povidone, povidone vinyl acetate, vitamin E, menthol, dimethyl isosorbide, acetone, ethanol, tetrafluroroethane and, dichlorodifluoromethane.

U.S. Patent No. 4,393,073 also suggests vitamin E as an active ingredient in pharmaceutical compositions containing ethanol.

WO 00/01351 discloses pharmaceutical compositions for the transdermal administration of a medicament or other active agent by topical application of the composition to the skin of humans or other animals are described. Methodology for formulating such compositions which provide for very rapid uptake of the medicament and transmigration into and through the skin to either fatty tissues or the vascular system, while minimizing irritation to the skin and/or immunological response, is based on a transdermal delivery system (TDS) wherein the medicament is modified to form a true solution in a complex formed from particular solvents and solvent and solute modifiers in combination with skin stabilizers. Uptake of the medicament is further facilitated and made more rapid by including Forskolin or other source of cellular energy, namely induction of cAMP or cGMP. Selection of specific solvents and solvent and solute modifiers and other functional ingredients and the amounts thereof are chosen such that there is a balance between the sum of the mole-moments [(molar amount of each individual ingredient) X (dipole moment of that ingredient)] of the delivery system and the sum of the molar moments of the composition in which the medicament is dissolved. Preferably, the van der Waals forces of the delivery system is also similarly matched to the van der Waals forces of the total composition, namely, delivery system plus active agent.

US 2004/0167152 discloses parenteral formulations of rapamycin 42-ester with 3-hydroxy-2-(hydroxymethyl)-2-methylpropionic acid (CCI-779).

WO 03/020318 discloses a topical w/o-emulsion composition for cosmetic or medical use, comprising an oil phase and an aqueous phase dispersed in the continuous oil phase. The oil phase contains at least one non-polar oil, monoglycosylceramide, and optionally ethanol. The composition is able to form a macroscopically homogeneous and stable w/o-emulsion.

US 2008/0045490 discloses compositions comprising formula 1 steroids, e.g., 16α-bromo-3β-hydroxy-5α-androstan-17-one hemihydrate and one or more excipients, typically wherein the composition comprises less than about 3% water. The compositions are useful to make improved pharmaceutical formulations. The disclosure also provides methods of intermittent dosing of steroid compounds such as analogs of 16a-bromo-3β-hydroxy-5α-androstan-17-one and compositions useful in such dosing regimens. The disclosure further provides compositions and methods to inhibit pathogen (viral) replication, ameliorate symptoms associated with immune dysregulation and to modulate immune responses in a subject using certain steroids and steroid analogs. The disclosure also provides methods to make and use these immunomodulatory compositions and formulations.

WO 03/057208 discloses taxane-based compostions and methods of using the same to achieve target blood levels of a taxane in a mammal, e.g., to treat taxane-responsive malignant and non-malignant diseases. Compositions of the disclosure exhibit long-term stability and overall palatability. Also disclosed are methods for using the compositions as analytical tools for pharmacokinetic studies.

US 2006/0287301 discloses formulations of fluphenzine HCl, derivatives thereof and other phenothiazines for the purpose of treating a mammal, preferably a human, in need thereof.

### Summary of the Invention

An aspect of the present invention relates to a pharmaceutical solution consisting of a therapeutic agent dissolved in alpha-tocopherol and ethanol and as claimed in the claims attached hereto.

Another aspect of the present invention relates to methods for producing these pharmaceutical solutions.

Another aspect of the present invention discloses methods of treatment of a patient with these pharmaceutical solutions.

### Detailed Description of the Invention

The present invention is directed to the use of alpha-tocopherols and ethanol as pharmaceutically acceptable solvents for solubilizing hydrophobic or lipophilic therapeutic agents and as claimed in the claims attached hereto. Advantageously, the resulting pharmaceutical solution is not an emulsion or vesicle, and can be used directly in the production of pharmaceutical compositions. Moreover, the combination of a tocopherol and/or a tocotrienol and an alcohol and/or glycol is much less irritating to the skin and/or mucous membranes than pure alcohol solutions and generally provides higher loading of a therapeutic agent than emulsions, liposomes, encapsulations, or cyclodextrins.

A solution in the context of the present invention is a homogeneous mixture composed of three or more substances. In such a mixture, a solute is dissolved in another substance, known as a solvent. In accordance with the present invention, a pharmaceutical solution is formed by dissolving a therapeutic agent in an alpha-tocopherol and ethanol as solvents. The therapeutic agent is dissolved completely in the tocopherol and/or a tocotrienol and the alcohol and/or glycol solvents, as claimed in the claims attached hereto. The resulting pharmaceutical solutions can be used in a variety of pharmaceutical compositions with various modes of administration.

The combination of tocopherol and/or a tocotrienol and alcohol and/or glycol is also useful in solubilizing at least in part amphiphobic therapeutic agents. In this embodiment, the solution acts as a transport phase through partial solubilization to increase the bioavailability of the amphiphobic therapeutic agent from a finely divided suspension of the agent.

Tocopherols and/or tocotrienols include a family of natural and synthetic compounds, also known by the generic names tocols or vitamin E. Alpha-tocopherol is the most abundant and active form of this family of compounds, and it has the following chemical structure:

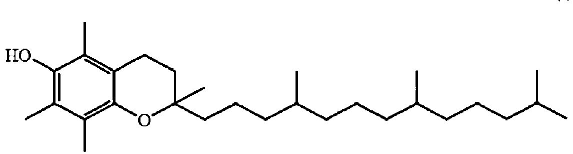

Other members of this family include beta-, gamma-, and delta-tocopherol, alpha-, beta-, gamma-, and delta-tocotrienols, tocopsoralen, alpha-tocopherol derivatives and/or analogs such as tocopherol acetate, phosphate, succinate, nicotinate and linoleate, as well as isomers thereof and esters thereof. The tocopherol(s) and/or tocotrienol(s) employed is alpha-tocopherol.

Examples of alcohol(s) include, but are not limited to ethanol, propyl alcohol, butyl alcohol, pentanol, benzyl alcohol, and any isomers thereof, and any combinations thereof. Examples of glycols include, but are not limited to ethylene glycol, propylene glycol, butylene glycol, pentylene glycol, and any isomers thereof, and any combinations thereof. The alcohol is ethanol (ethyl alcohol). Preferred is use of an ethanol that is biocompatible in the sense that it is not toxic and does not cause any physiological or pharmacological effects. In this regard, the ethanol is desirably 180 to 200 proof ethanol, *i.e*., in the range of 90-100% ethanol. Advantageously, diluting a tocopherol or tocotrienol with an alcohol or glycol dramatically reduces the inherent viscosity of the tocopherol or tocotrienol thereby allowing for generation of sprayable formulations.

In accordance with the present invention, solutions of one or more tocopherols and/or tocotrienols and one or more alcohols and/or glycols are used in the solubilization of hydrophobic or lipophilic therapeutic agents thereby providing increased bioavailability of the therapeutic agent.

As a non-limiting example, the solubility of Diazepam at room temperature is less than or equal to 6.67% in 190 proof ethanol. However, combining tocopherol and ethanol has been found to provide solubility of the Diazepam approaching the 10% level. By way of illustration, at 70% tocopherol:30% ethanol (200 proof), Diazepam is soluble to greater than or equal to 8% and at 95% tocopherol:5% ethanol (200 proof), Diazepam is soluble at greater than or equal to 9%.

Accordingly, alpha-tocopherol and ethanol constitute 60% to 99% and 1% to 40%, respectively, of the pharmaceutical solution. In other embodiments, the alpha-tocopherol and ethanol constitute approximately 70% to 90% and 10% to 30%, respectively, of the pharmaceutical solution. In still other embodiments, the tocopherol and ethanol are used at ratios of approximately 95:5, 90:10, 85:15, 80-20, 75:25, 70:30, 65:35, or 60:40, respectively.

Pharmaceutical solutions of the present invention can be produced by dissolving any difficult to solubilize therapeutic agent (*i.e*., hydrophobic or lipophilic therapeutic agents) in one or more tocopherols and/or tocotrienols and one or more alcohols and/or glycols as pharmaceutically acceptable solvents. By therapeutic agents it is meant to be inclusive of, but is not limited to, small organic molecules, therapeutic peptides, non-peptides and nucleotides. Hydrophobic derivatives of water-soluble molecules such as lipid conjugates/prodrugs are also within the scope of therapeutic agents.

Exemplary hydrophobic or lipophilic therapeutic agents which can be solubilized in accordance with the present invention include, but are in no way limited to, steroids such as Dexamethasone, 17-beta-Estradiol; benzodiazepenes such as Diazepam, alpraxolam, bromazepam, chlordiazepoxidem, clonazepam, estazolam, flunitrazepam, flurazepam, lorazepam, lormetazepam, mexazolam, nitrazepam, oxazepam, temazepam, and triazolam; Rapamycin and analogues; Taxol (paclitaxel) and analogues; Actinomycin D; Prostaglandins (PGE1); Vitamin A; Probucol; Batimastat; Statins (HMG-CoA Reductase Inhibitors; Trapidil (and other anti-proliferative Growth Factor Inhibitors); Cytochalasin B; and microtubule binding agents such as epothilones, elutherobin and discodermolide. Pharmaceutical solutions and compositions formulated from the solutions may comprise one or more therapeutic agents in solution. Further, pharmaceutical compositions formulated from the pharmaceutical solutions of the present invention may further comprise one or more additional therapeutic agents in encapsulated or micronized (not dissolved) forms.

The present invention also discloses the use of a combination of tocopherol and/or a tocotrienol and alcohol and/or glycol to solubilize at least in part amphiphobic therapeutic agents. In this disclosure, the solution acts as a transport phase through partial solubilization to increase the bioavailability of the amphiphobic therapeutic agent from a finely divided suspension of the agent.

Pharmaceutical solutions of the invention can be formulated into pharmaceutical compositions for administration to animals, preferably humans, via intravascular, oral, intramuscular, cutaneous and subcutaneous routes. Specifically, pharmaceutical compositions of the present invention can be administered by any of the following nonlimiting exemplary routes, intraabdominal, intraarterial, intraarticular, intracapsular, intracervical, intracranial, intraductal, intradural, intralesional, intralocular, intralumbar, intramural, intranasal, intraocular, intraoperative, intraparietal, intraperitoneal, intrapleural, intrapulmonary, intraspinal, intrathoracic, intratracheal, intratympanic, intrauterine, and intraventricular. The pharmaceutical compositions of the present invention can be nebulized using mechanical nebulizers or suitable aerosol propellants which are known in the art for pulmonary delivery of lipophilic compounds. The most suitable route in any given case will depend on the nature and severity of the condition being treated and on the nature of the particular therapeutic agent which is being used.

Pharmaceutical solutions of the instant invention are particularly useful in formulations to be administered to mucosal membranes, i.e. the nasal mucosa or lungs of a subject by any suitable means. For many therapeutic agents, administration via the nasal route provides for faster attainment of therapeutic levels of the therapeutic agent systemically. However, many therapeutic agents are so slightly soluble in water that a therapeutically effective amount cannot be dissolved in a volume of aqueous solvent that is amenable to application to a mucosal membrane. Use of a pharmaceutical solution of the present invention, however, provides for improved ability to dissolve hydrophilic and lipophilic therapeutic agents, thus providing a useful delivery system for administration of such agents to one or more mucosal membranes, including the nasal mucosal membranes. Such solutions can be administered via, for example, a metered dose inhaler or nebulizer, or in a mist sprayer.

The instant pharmaceutical solutions comprising a therapeutic agent, one or more tocopherols and/or tocotrienols and one or more alcohols and/or glycols can also be formulated into a pharmaceutical composition for injection by combining the instant pharmaceutical solution with, *e.g*., saline solution or water and a Vitamin E solubilizing agent such as Cremophor. Such pharmaceutical compositions may further contain other pharmaceutically acceptable additives such as, but not limited to, acidifying, alkalizing, buffering, chelating, complexing and solubilizing agents, antioxidants and antimicrobial preservatives, penetration enhancers, humectants, suspending and/or viscosity modifying agents, tonicity and wetting or other biocompatible materials.

For oral therapeutic administration, the instant pharmaceutical solutions can be combined with one or more carriers and used in the form of ingestible tablets, buccal tablets, troches, capsules, elixirs, suspensions, syrups, wafers, chewing gums, foods and the like. Such compositions and preparations should contain at least 0.1% of active compound. Tablets, troches, pills, capsules, and the like can also contain one or more of the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring. The above listing is merely representative and one skilled in the art could envision other binders, excipients, sweetening agents and the like. When the unit dosage form is a capsule, it can contain, in addition to materials of the above type, a liquid carrier, such as a vegetable oil or a polyethylene glycol. Various other materials can be present as coatings or to otherwise modify the physical form of the solid unit dosage form. For instance, tablets, pills, or capsules can be coated with gelatin, wax, shellac or sugar and the like.

A syrup or elixir can contain the instant pharmaceutical solution, sucrose or fructose as a sweetening agent, methyl and propylparabens as preservatives, a dye and flavoring such as cherry or orange flavor. Of course, any material used in preparing any unit dosage form should be substantially non-toxic in the amounts employed.

In addition, the instant pharmaceutical solution can be formulated into sustained-release preparations and devices including, but not limited to, those relying on osmotic pressures to obtain a desired release profile.

Formulations suitable for parenteral administration can be aqueous or non-aqueous injection solutions, which are generally isotonic with the blood of the intended recipient. These preparations can contain anti-oxidants, buffers, bacteriostats and solutes which render the formulation isotonic with the blood of the intended recipient. Aqueous and non-aqueous sterile suspensions can include suspending agents and thickening agents. The formulations can be presented in unit\dose or multi-dose containers, for example sealed ampoules and vials.

Formulations suitable for topical application to the skin can take the form of an ointment, cream, lotion, paste, gel, spray, aerosol, oil or other pharmaceutical formulation which accomplishes direct contact between the therapeutic agent and the skin. Topical formulations can also be prepared which are suitable for occlusive therapy.

Formulations in the forms of ointments, creams, lotions and pastes can generally have carriers in the forms of oleaginous bases (*e.g*., White Petrolatum and White Ointment); absorption bases formed by adding a water-in-oil emulsifying agent to an oleaginous base (*e.g*., Hydrophilic Petrolatum, AQUABASE, and AQUAPHOR); water-in-oil emulsion bases, prepared by adding water to an absorption base (*e.g.*, HYDROCREAM, EUCERIN, NIVEA, and Cold Cream); oil-in-water emulsion bases (*e.g*., DERMABASE, UNIBASE, VELVACHOL, and hydrophilic ointment); and water soluble bases (*e.g*., polyethylene glycol ointment such as PEG 400-600 G or PEG 3350-400 G). Suitable carriers to produce a spray, gel, or aerosol are well-known in the art.

A carrier for topical application can also contain additional ingredients such as other carriers, moisturizers, penetration enhancers, humectants, emollients, dispersants, radiation blocking compounds, cleansing agents, anti-infective agents (*e.g*., antibiotics, fungicides, scabicides, or pediculicides), anti-inflammatory agents (*e.g*., corticosteroids), keratolytics (agents that soften, loosen, and facilitate exfoliation of the squamous cells of the epidermis), as well as other suitable materials that do not have a significant adverse effect on the activity of the topical composition. Additional ingredients can include, for example a sodium acid phosphate moisturizer, witch hazel extract, glycerine humectant, apricot kernal oil emollient, or corn oil dispersant. Other materials which can optionally be included in a topical composition include inositol or B-complex vitamins.

Formulations suitable for transdermal administration can be presented as discrete patches adapted to remain in intimate contact with the epidermis of the recipient for a prolonged period of time. Formulations suitable for transdermal administration can also be delivered by iontophoresis (*see*, for example, Pharmaceutical Research 3 (6):318 (1986)) and typically take the form of an optionally buffered aqueous solution. Formulations of the present invention are also suitable for delivery via microneedle delivery technology for cutaneous administration.

## Claims

1. A pharmaceutical solution consisting of a therapeutic agent dissolved in alpha-tocopherol and ethanol, wherein the therapeutic agent is hydrophobic or lipophilic; and
wherein the alpha-tocopherol and the ethanol constitute 60% to 99% and 1% to 40%, respectively, of the pharmaceutical solution, wherein the hydrophobic or lipophilic therapeutic agent is Diazepam.

2. The pharmaceutical solution of claim 1, wherein the tocopherol and the ethanol are used at ratios of approximately 95:5, 90:10, 85:15, 80-20, 75:25, 70:30, 65:35, or 60:40, respectively.

3. A pharmaceutical composition comprising the pharmaceutical solution of either of claims 1 or 2.

4. The pharmaceutical composition of claim 3 further comprising one or more additional therapeutic agents in encapsulated or micronized forms.

5. A method for solubilizing a therapeutic agent comprising dissolving a therapeutic agent in alpha-tocopherol and ethanol to form a pharmaceutical solution consisting of the therapeutic agent dissolved in alpha-tocopherol and ethanol, wherein the therapeutic agent is hydrophobic or lipophilic; and
wherein the alpha-tocopherol and the ethanol constitute 60% to 99% and 1% to 40%, respectively, of the pharmaceutical solution, wherein the hydrophobic or lipophilic therapeutic agent is Diazepam.

6. The method of claim 5 wherein the tocopherol and the ethanol are used at ratios of approximately 95:5, 90:10, 85:15, 80-20, 75:25, 70:30, 65:35, or 60:40, respectively.

## Patentansprüche

1. Pharmazeutische Lösung, die aus einem Therapeutikum besteht, das in alpha-Tocopherol und Ethanol gelöst ist, wobei das Therapeutikum hydrophob oder lipophil ist; und wobei das alpha-Tocopherol und das Ethanol 60 % bis 99 % bzw. 1 % bis 40 % der pharmazeutischen Lösung darstellen, wobei das hydrophobe oder lipophile Therapeutikum Diazepam ist.

2. Pharmazeutische Lösung nach Anspruch 1, wobei das Tocopherol und das Ethanol in Verhältnissen von ungefähr 95 : 5, 90 : 10, 85 : 15, 80 : 20, 75 : 25, 70 : 30, 65 : 35 bzw. 60 : 40 verwendet werden.

3. Pharmazeutische Zusammensetzung, die die pharmazeutische Lösung nach einem der Ansprüche 1 oder 2 umfasst.

4. Pharmazeutische Zusammensetzung nach Anspruch 3, die weiter ein oder mehrere zusätzliche Therapeutika in verkapselten oder mikronisierten Formen umfasst.

5. Verfahren für die Solubilisierung eines Therapeutikums, das das Lösen eines Therapeutikums in alpha-Tocopherol und Ethanol umfasst, um eine pharmazeutische Lösung zu bilden, die aus dem Therapeutikum besteht, das in alpha-Tocopherol und Ethanol gelöst ist,
wobei das Therapeutikum hydrophob oder lipophil ist; und
wobei das alpha-Tocopherol und das Ethanol 60 % bis 99 % bzw. 1 % bis 40 % der pharmazeutischen Lösung darstellen, wobei das hydrophobe oder lipophile Therapeutikum Diazepam ist.

6. Verfahren nach Anspruch 5, wobei das Tocopherol und das Ethanol in Verhältnissen von ungefähr 95 : 5, 90 : 10, 85 :15, 80 : 20, 75 : 25, 70 : 30, 65 : 35 bzw. 60 : 40 verwendet werden.

## Revendications

1. Solution pharmaceutique constituée d'un agent thérapeutique solubilisé dans de l'alpha-tocophérol et de l'éthanol, dans laquelle l'agent thérapeutique est hydrophobe ou lipophile ; et où l'alpha-tocophérol et de l'éthanol constituent de 60% à 99% et de 1% à 40%, respectivement, de la solution pharmaceutique, où l'agent thérapeutique hydrophobe ou lipophile est le diazépam.

2. Solution pharmaceutique selon la revendication 1, dans laquelle le tocophérol et l'éthanol sont utilisés selon des rapports d'environ 95:5, 90:10, 85:15, 80:20, 75:25, 70:30, 65:35 ou 60:40, respectivement.

3. Composition pharmaceutique comprenant la solution pharmaceutique selon l'une ou l'autre parmi les revendications 1 et 2.

4. Composition pharmaceutique selon la revendication 3, comprenant en outre un ou plusieurs agents thérapeutiques supplémentaires sous des formes encapsulées ou micronisées.

5. Méthode de solubilisation d'un agent thérapeutique, comprenant la solubilisation d'un agent thérapeutique dans de l'alpha-tocophérol et de l'éthanol afin de former une solution pharmaceutique constituée de l'agent thérapeutique solubilisé dans de l'alpha-tocophérol et de l'éthanol, où l'agent thérapeutique est hydrophobe ou lipophile ; et
où l'alpha-tocophérol et de l'éthanol constituent de 60% à 99% et de 1% à 40%, respectivement, de la solution pharmaceutique, où l'agent thérapeutique hydrophobe ou lipophile est le diazépam.

6. Méthode selon la revendication 5, dans laquelle le tocophérol et l'éthanol sont utilisés selon des rapports d'environ 95:5, 90:10, 85:15, 80:20, 75:25, 70:30, 65:35 ou 60:40, respectivement.
